# EUROPEAN PATENT APPLICATION

(11) **EP 2 251 007 A2**
(43) Date of publication of application: **17.11.2010**
(21) Application number: 10173048.9
(22) Date of filing: 23.09.2003
(51) Int. Cl.: A61K 31/135, A61K 38/21, A61P 25/00

(54) **Sphingosine-1-phosphate (S1P) receptor agonists for use in the treatment of demyelinating diseases**

(30) Priority: 24.09.2002 US 413172 P; 07.07.2003 US 485132 P
(62) Divisional of application: 03798176.8
(71) Applicant: Novartis AG, 4056 Basel (CH); Novartis Pharma GmbH, 1230 Wien (AT)
(72) Inventor: Foster, Carolyn Ann, 1130, Wien (AT); Hiestand, Peter C., 4123, Allschwil (CH); Glue, Paul William, Flemington, NJ 08822 (US)
(74) Representative: Rouquayrol, Céline Hélène

(57) **Abstract**

Disclosed are pharmaceutical combinations comprising at least one S1P receptor agonist, as well as a method for treating demyelinating diseases, e.g. multiple sclerosis or disorders associated therewith or Guillain-Barré syndrome, comprising co-administration, e.g. concomitantly or in sequence, of a therapeutically effective amount of a) an S1P receptor agonist, and b) at least one co-agent shown to have clinical activity against at least one symptom of a demyelinating disease.

## Description

The present invention relates to pharmaceutical combinations comprising at least one S1 P receptor agonist and their uses in treating demyelinating diseases, e.g. multiple sclerosis and disorders associated therewith.

Multiple sclerosis is an immune-mediated disease of the central nervous system white matter with chronic inflammatory demyelination leading to progressive decline of motor and sensory functions and permanent disability. Manifestations of clinical disease usually begin in early adulthood, with women outnumbering men 2:1. The therapy of multiple sclerosis is only partially effective, and in most cases only offers a delay in disease progression despite anti-inflammatory and immunosuppressive treatment. Clinicians usually categorize patients into four types of disease patterns:
- *Relapsing-remitting* (RR-MS): Discrete motor, sensory, cerebellar or visual attacks that occur over 1-2 weeks and often resolve over 1-2 months, with or without treatment. Some patients accrue disability with each episode, yet remain clinically stable between relapses. About 85% of patients initially experience the RR form of MS, but within 10 years about half will develop the secondary progressive form.
- *Secondary-progressive* (SP-MS): Initially RR followed by gradually increasing disability, with or without relapses. Major irreversible disabilities appear most often during SP.
- *Primary-progressive* (PP-MS): Progression disease course from onset without any relapses or remissions, affecting about 15% of MS patients.
- *Progressive-relapsing* (PR-MS): Progressive disease from onset with clear acute relapses; periods between relapses characterized by continuing progression.

Accordingly, there is a need for agents which are effective in the treatment of demyelinating diseases, e.g. multiple sclerosis or Guillain-Barré syndrome, e.g. including reduction of, alleviation of, stabilization of or relief from the symptoms or illness which affect the organism.

It has now been found that a combination comprising at least one S1 P receptor agonist and a co-agent, e.g. as defined below, has a beneficial effect on demyelinating diseases, e.g. multiple sclerosis and the disorders associated therewith.

In accordance with the particular findings of the present invention, there is provided
1. A pharmaceutical combination comprising:
   a) an S1 P receptor agonist, and
   b) at least one co-agent shown to have clinical activity against at least one demyelinating disease symptom, e.g. a multiple sclerosis symptom or a symptom of Guillain-Barré syndrome.
2. 1 A method for treating a demyelinating disease, e.g. multiple sclerosis or disorders associated therewith or Guillain-Barré syndrome, comprising co-administration, e.g. concomitantly or in sequence, of a therapeutically effective amount of an S1 P receptor agonist, e.g. a compound of formulae I to VII as defined hereinafter, and at least one co-agent, e.g. as indicated hereinafter.
2.2 A method for alleviating or delaying progression of the symptoms of a demyelinating disease, e.g. multiple sclerosis or Guillain-Barré syndrome, comprising co-administration, e.g. concomitantly or in sequence, of a therapeutically effective amount of an S1 P receptor agonist, e.g. a compound of formulae I to VII as defined herein after, and at least one co-agent, e.g. as indicated hereinafter.
   An early symptom of multiple sclerosis is optic neuritis. Accordingly, the present invention also provides
2.3 A method for treating, alleviating or delaying progression of optic neuritis in a subject in need thereof, comprising administering to said subject a therapeutically effective amount of an S1 P receptor agonist, e.g. a compound of formulae I to VII as specified herein after, e.g. Compound A or B or a pharmaceutically acceptable salt thereof.
3. A pharmaceutical combination as disclosed herein for use in any one of the methods 2.1 to 2.3.
4.1 A pharmaceutical composition for treating, alleviating or delaying progression of optic neuritis comprising an S1 P receptor agonist, e.g. a compound of formulae I to VII as defined herein after, e.g. Compound A or B, together with one or more pharmaceutically acceptable diluents or carriers therefor.
4.2 A compound of formulae I to VII as defined herein after, e.g. Compound A or B, for use in the treatment, alleviating or delay of progression of optic neuritis.
4.3 An S1P receptor agonist, e.g. a compound of formulae I to VII as defined herein after, e.g. Compound A or B, for use in the preparation of a medicament for use in the treatment, alleviating or delay of progression of optic neuritis.
5.1 Use of an S1P receptor agonist, e.g. a compound of formulae I to VII as defined herein after, e.g. Compound A or B, for the preparation of a medicament for treating, alleviating or delaying the progression of optic neuritis.
5.2 Use of a) a sphingosine-1-phosphate (S1P) receptor agonist, and b) at least one co-agent shown to have clinical activity against at least one symptom of a demyelinating disease, for the preparation of a pharmaceutical combination for treating, alleviating or delaying progression of the symptoms of a demyelinating disease, e.g. for the preparation of a pharmaceutical combination for separate, simultaneous or sequential use in such a method.
5.3 A pharmaceutical composition as disclosed herein for separate, simultaneous or sequential use in medicine, e.g. in a method as disclosed at 2.1 to 2.3.

The term "pharmaceutical combination" as used herein means a product that results from the mixing or combining of more than one active ingredient and includes both fixed and non-fixed combinations of the active ingredients.

The term "fixed combination" as that term is used herein means that the active ingredients, e.g. the S1 P receptor agonist and a co-agent, are both administered to a patient simultaneously in the form of a single entity or dosage. As an example, a fixed combination would be one capsule containing two active ingredients.

The term "non-fixed combination" as that term is used herein means that the active ingredients, e.g. the S1 P receptor agonist and a co-agent, are both administered to a patient as separate entities either simultaneously, concurrently or sequentially with no specific time limits, wherein such administration provides therapeutically effective levels of the two compounds in the body, preferably at the same time. As an example, a non-fixed combination would be two capsules each containing one active ingredient where the purpose is to have the patient achieve treatment with both active ingredients together in the body.

An S1 P receptor agonist is an immunomodulating compound which elicits a lymphopenia resulting from a re-distribution, preferably reversible, of lymphocytes from circulation to secondary lymphatic tissue, without evoking a generalized immunosuppression. Naïve cells are sequestered; CD4 and CD8 T-cells and B-cells from the blood are stimulated to migrate into lymph nodes (LN) and Peyer's patches (PP), and thus for example infiltration of cells into transplanted organs is inhibited.

Examples of appropriate S1 P receptor agonists are, for example:
- Compounds as disclosed in EP627406A1, e.g. a compound of formula I 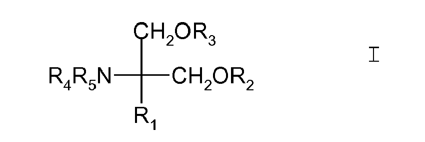 wherein R₁ is a straight- or branched (C₁₂₋₂₂)carbon chain
   - which may have in the chain a bond or a hetero atom selected from a double bond, a triple bond, O, S, NR₆, wherein R₆ is H, alkyl, aralkyl, acyl or alkoxycarbonyl, and carbonyl, and/or
   - which may have as a substituent alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, amino, hydroxyimino, hydroxy or carboxy; or
   R₁ is
   - a phenylalkyl wherein alkyl is a straight- or branched (C₆₋₂₀)carbon chain; or
   - a phenylalkyl wherein alkyl is a straight- or branched (C₁₋₃₀)carbon chain wherein said phenylalkyl is substituted by
   - a straight- or branched (C₆₋₂₀)carbon chain optionally substituted by halogen,
   - a straight- or branched (C₆₋₂₀)alkoxy chain optionally substitued by halogen,
   - a straight- or branched (C₆₋₂₀)alkenyloxy,
   - phenylalkoxy, halophenylalkoxy, phenylalkoxyalkyl, phenoxyalkoxy or phenoxyalkyl,
   - cycloalkylalkyl substituted by C₆₋₂₀alkyl,
   - heteroarylalkyl substituted by C₆₋₂₀alkyl,
   - heterocyclic C₆₋₂₀alkyl or
   - heterocyclic alkyl substituted by C₂₋₂₀alkyl,
   and wherein
   the alkyl moiety may have
   - in the carbon chain, a bond or a heteroatom selected from a double bond, a triple bond, O, S, sulfinyl, sulfonyl, or NR₆, wherein R₆ is as defined above, and
   - as a substituent alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, amino, hydroxy or carboxy, and
   each of R₂, R₃, R₄ and R₅, independently, is H, C₁₋₄alkyl or acyl or a pharmaceutically acceptable salt thereof;
- Compounds as disclosed in EP 1002792A1, e.g. a compound of formula II 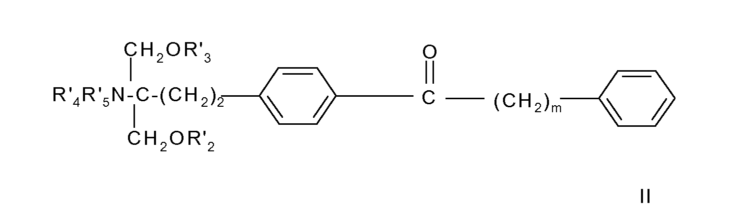 wherein m is 1 to 9 and each of R'₂, R'₃, R'₄ and R'₅, independently, is H, alkyl or acyl,
   or a pharmaceutically acceptable salt thereof;
- Compounds as disclosed in EP0778263 A1, e.g. a compound of formula III 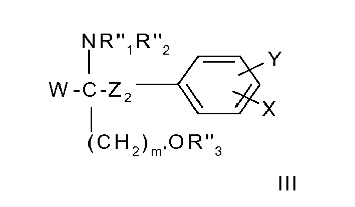 wherein W is H; C₁₋₆alkyl, C₂₋₆alkenyl or C₂₋₆alkynyl; unsubstituted or by OH substituted phenyl; R"₄O(CH₂)ₙ; or C₁₋₆alkyl substituted by 1 to 3 substituents selected from the group consisting of halogen, C₃₋₈cycloalkyl, phenyl and phenyl substituted by OH;
   X is H or unsubstituted or substituted straight chain alkyl having a number p of carbon atoms or unsubstituted or substituted straight chain alkoxy having a number (p-1) of carbon atoms, e.g. substituted by 1 to 3 substitutents selected from the group consisting of C₁₋₆ alkyl, OH, C₁₋₆alkoxy, acyloxy, amino, C₁₋₆alkylamino, acylamino, oxo, haloC₁₋₆alkyl, halogen, unsubstituted phenyl and phenyl substituted by 1 to 3 substituents selected from the group consisting of C₁₋₆alkyl, OH, C₁₋₆alkoxy, acyl, acyloxy, amino, C₁₋₆alkylamino, acylamino, haloC₁₋₆alkyl and halogen; Y is H, C₁₋₆alkyl, OH, C₁₋₆alkoxy, acyl, acyloxy, amino, C₁₋₆alkylamino, acylamino, haloC₁₋₆alkyl or halogen, Z₂ is a single bond or a straight chain alkylene having a number or carbon atoms of q,
   each of p and q, independently, is an integer of 1 to 20, with the proviso of 6≤p+q≤23, m' is 1, 2 or 3, n is 2 or 3,
   each of R"₁, R"₂, R"₃ and R"₄, independently, is H, C₁₋₄alkyl or acyl,
   or a pharmaceutically acceptable salt thereof,
- Compounds as disclosed in WO02/18395, e.g. a compound of formula IVa or IVb 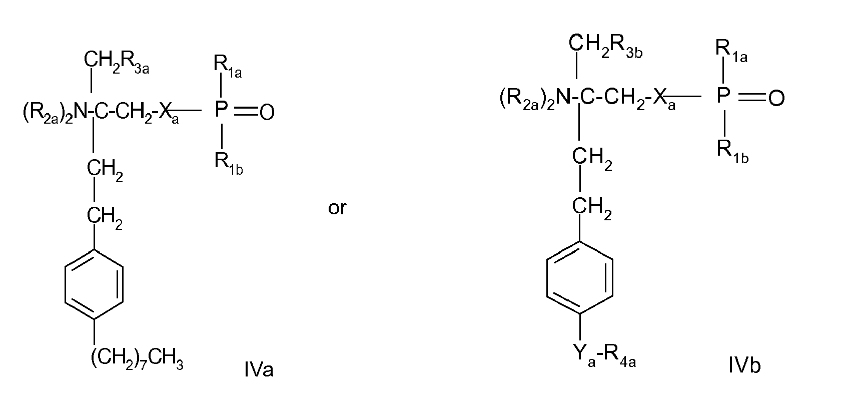 wherein Xₐ is O, S, NR₁ₛ or a group -(CH₂)ₙₐ-, which group is unsubstituted or substituted by 1 to 4 halogen; nₐ is 1 or 2, R₁ₛ is H or (C₁₋₄)alkyl, which alkyl is unsubstituted or substituted by halogen; R₁ₐ is H, OH, (C₁₋₄)alkyl or O(C₁₋₄)alkyl wherein alkyl is unsubstituted or substituted by 1 to 3 halogen; R_{1b} is H, OH or (C₁₋₄)alkyl, wherein alkyl is unsubstituted or substituted by halogen; each R₂ₐ is independently selected from H or (C₁₋₄)alkyl, which alkyl is unsubstituted or substitued by halogen; R₃ₐ is H, OH, halogen or O(C₁₋₄)alkyl wherein alkyl is unsubstituted or substituted by halogen; and R_{3b} is H, OH, halogen, (C₁₋₄)alkyl wherein alkyl is unsubstituted or substituted by hydroxy, or O(C₁₋₄)alkyl wherein alkyl is unsubstituted or substituted by halogen; Yₐ is -CH₂-, -C(O)-, -CH(OH)-, - C(=NOH)-, O or S, and R₄ₐ is (C₄₋₁₄)alkyl or (C₄₋₁₄)alkenyl;
   or a pharmaceutically acceptable salt or hydrate thereof;
   - Compounds as disclosed in WO 02/076995, e.g. a compound of formula V 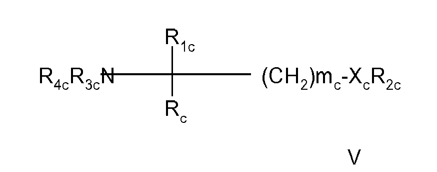 wherein
   - m_{c}: is 1, 2 or 3;
   - X_{c}: is O or a direct bond;
   - R_{1c}: is H; C₁₋₆alkyl optionally substituted by OH, acyl, halogen, C₃₋₁₀cycloalkyl, phenyl or hydroxy-phenylene; C₂₋₆alkenyl; C₂₋₆alkynyl; or phenyl optionally substituted by OH;
   - R_{2c}: is 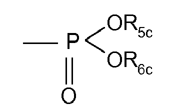
   wherein R_{5c} is H or C₁₋₄alkyl optionally substituted by 1, 2 or 3 halogen atoms, and R_{6c} is H or C₁₋₄alkyl optionally substituted by halogen;
   each of R_{3c} and R_{4c}, independently, is H, C₁₋₄alkyl optionally substituted by halogen, or acyl, and
   - R_{c}: is C₁₃₋₂₀alkyl which may optionally have in the chain an oxygen atom and which may optionally be substituted by nitro, halogen, amino, hydroxy or carboxy; or a residue of formula (a) 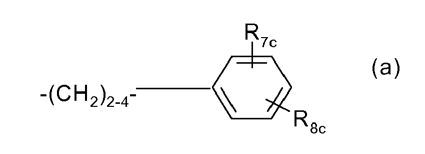 wherein R_{7c} is H, C₁₋₄alkyl or C₁₋₄alkoxy, and R_{8c} is substituted C₁₋₂₀alkanoyl, phenylC₁₋₁₄alkyl wherein the C₁₋₁₄alkyl is optionally substituted by halogen or OH, cycloalkylC₁₋₁₄alkoxy or phenylC₁₋₁₄alkoxy wherein the cycloalkyl or phenyl ring is optionally substituted by halogen, C₁₋₄alkyl and/or C₁₋₄alkoxy, phenylC₁₋₁₄-alkoxy-
   C₁₋₁₄alkyl, phenoxyC₁₋₁₄alkoxy or phenoxyC₁₋₁₄alkyl,
   - R_{c}: being also a residue of formula (a) wherein R_{8c} is C₁₋₁₄alkoxy when R_{1c} is C₁₋₄-alkyl,
   C₂₋₆alkenyl or C₂₋₆alkynyl, or a compound of formula VI 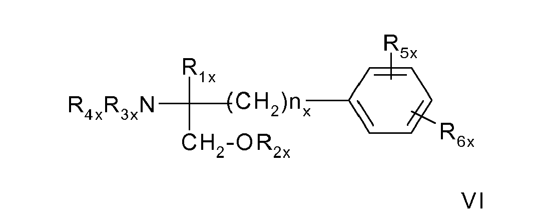 wherein
   - nₓ: is 2, 3 or 4
   - R₁ₓ: is H; C₁₋₆alkyl optionally substituted by OH, acyl, halogen, cycloalkyl, phenyl or hydroxy-phenylene; C₂₋₆alkenyl; C₂₋₆alkynyl; or phenyl optionally substituted by OH;
   - R₂ₓ: is H, C₁₋₄alkyl or acyl each of R₃ₓ and R₄ₓ, independently is H, C₁₋₄alkyl optionally substituted by halogen or acyl,
   - R₅ₓ: is H, C₁₋₄alkyl or C₁₋₄alkoxy, and
   - R₆ₓ: is C₁₋₂₀alkanoyl substituted by cycloalkyl; cyloalkylC₁₋₁₄alkoxy wherein the cycloalkyl ring is optionally substituted by halogen, C₁₋₄alkyl and/or C₁₋₄alkoxy; phenylC₁₋₁₄alkoxy wherein the phenyl ring is optionally substituted by halogen, C₁₋₄alkyl and/or C₁₋₄alkoxy,
   - R₆ₓ: being also C₄₋₁₄alkoxy when R₁ₓ is C₂₋₄alkyl substituted by OH, or pentyloxy or hexyloxy when R₁ₓ is C₁₋₄akyl,
   provided that R₆ₓ is other than phenyl-butylenoxy when either R₅ₓ is H or R₁ₓ is methyl,
   or a pharmaceutically acceptable salt thereof;
- Compounds as disclosed in WO02/06268Al, e.g. a compound of formula VII 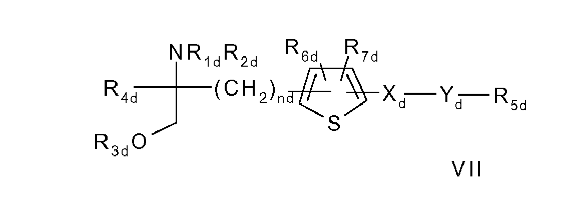 wherein each of R_{1d} and R_{2d}, independently, is H or an amino-protecting group; R_{3d} is hydrogen or a hydroxy-protecting group;
   R_{4d} is lower alkyl;
   n_{d} is an integer of 1 to 6;
   X_{d} is ethylene, vinylene, ethynylene, a group having a formula - D-CH₂- (wherein D is carbonyl, - CH(OH)-, O, S or N), aryl or aryl substituted by up to three substitutents selected from group a as defined hereinafter;
   Y_{d} is single bond, C₁₋₁₀alkylene, C₁₋₁₀alkylene which is substituted by up to three substitutents selected from groups a and b, C₁₋₁₀alkylene having O or S in the middle or end of the carbon chain, or C₁₋₁₀alkylene having O or S in the middle or end of the carbon chain which is substituted by up to three substituents selected from groups a and b;
   R_{5d} is hydrogen, cycloalkyl, aryl, heterocycle, cycloalkyl substituted by up to three substituents selected from groups a and b, aryl substituted by up to three substituents selected from groups a and b, or heterocycle substituted by up to three substituents selected from groups a and b; and
   each of R_{6d} and R_{7d}, independently, is H or a substituent selected from group a; <group a > is halogen, lower alkyl, halogeno lower alkyl, lower alkoxy, lower alkylthio, carboxyl, lower alkoxycarbonyl, hydroxy, lower aliphatic acyl, amino, mono- lower alkylamino, di-lower alkylamino, lower aliphatic acylamino, cyano or nitro; <group b > is cycloalkyl, aryl, heterocycle, each being optionally substituted by up to three substituents selected from group a;
   with the proviso that when R_{5d} is hydrogen, Y_{d} is a either a single bond or linear C₁₋₁₀ alkylene, or a pharmacologically acceptable salt or ester thereof.
- Compounds as disclosed in JP-14316985 (JP2002316985), e.g. a compound of formula VIII: 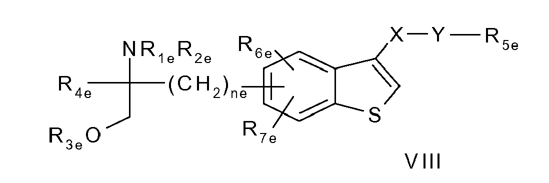 wherein R₁ₑ,R₂ₑ,R₃ₑ,R₄ₑ,R₅ₑ,R₆ₑ,R₇ₑ, nₑ, Xₑ and Yₑ are as disclosed in JP-14316985; or a pharmacologically acceptable salt or ester thereof.
- Compounds as disclosed in WO 03/29184 and WO 03/29205, e.g. compounds of formula IX 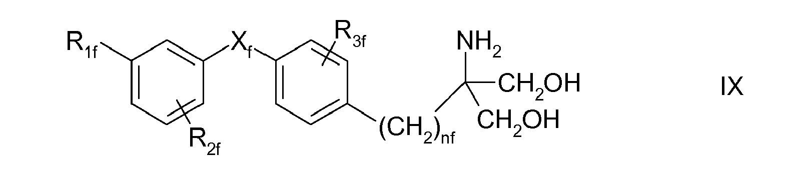 wherein X_{f} is O or S, and R_{1f}, R_{2f}, R_{3f} and n_{f} are as disclosed in WO 03/29184 and O3/29205, e.g. 2-amino-2-[4-(3-benzyloxyphenoxy)-2-chlorophenyl]propyl-1,3-propane-diol or 2-amino-2-[4-(benzyloxyphenylthio)-2- chlorophenyl]propyl-1,3-propane-diol.

In each case where citations of patent applications are given, the subject matter relating to the compounds is hereby incorporated into the present application by reference.

Acyl may be a residue R_{y}-CO- wherein Ry is C₁₋₆alkyl, C₃₋₆cycloalkyl, phenyl or phenyl-C₁₋₄alkyl. Unless otherwise stated, alkyl, alkoxy, alkenyl or alkynyl may be straight or branched.

When in the compounds of formula I the carbon chain as R₁ is substituted, it is preferably substituted by halogen, nitro, amino, hydroxy or carboxy. When the carbon chain is interrupted by an optionally substituted phenylene, the carbon chain is preferably unsubstituted. When the phenylene moiety is substituted, it is preferably substituted by halogen, nitro, amino, methoxy, hydroxy or carboxy.

Preferred compounds of formula I are those wherein R₁ is C₁₃₋₂₀alkyl, optionally substituted by nitro, halogen, amino, hydroxy or carboxy, and, more preferably those wherein R₁ is phenylalkyl substituted by C₆₋₁₄-alkyl chain optionally substituted by halogen and the alkyl moiety is a C₁₋₆alkyl optionally substituted by hydroxy. More preferably, R₁ is phenyl-C₁₋₆alkyl substituted on the phenyl by a straight or branched, preferably straight, C₆₋₁₄alkyl chain. The C₆₋₁₄alkyl chain may be in ortho, meta or para, preferably in para.

Preferably each of R₂ to R₅ is H.

A preferred compound of formula I is 2-amino-2-tetradecyl-1,3-propanediol. A particularly preferred S1 P receptor agonist of formula I is FTY720, i.e. 2-amino-2-[2-(4-octylphenyl) ethyl]propane-1 ,3-diol in free form or in a pharmaceutically acceptable salt form (referred to hereinafter as Compound A), e.g. the hydrochloride, as shown:

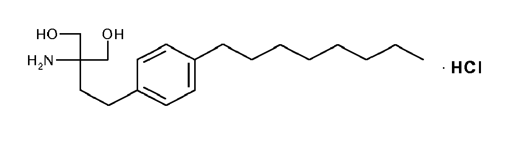

A preferred compound of formula II is the one wherein each of R'₂ to R'₅ is H and m is 4, i.e. 2-am!no-2-{2-[4-(1-oxo-5-phenylpentyl)phenyl]ethyl}propane-1,3-diol, in free form or in pharmaceutically acceptable salt form (referred to hereinafter as Compound B), e.g the hydrochloride.

A preferred compound of formula III is the one wherein W is CH₃, each of R"₁ to R"₃ is H, Z₂ is ethylene, X is heptyloxy and Y is H, i.e. 2-amino-4-(4-heptyloxyphenyl)-2-methyl-butanol, in free form or in pharmaceutically acceptable salt form (referred to hereinafter as Compound C), e.g. the hydrochloride. The R-enantiomer is particularly preferred.

A preferred compound of formula IVa is the FTY720-phosphate (R₂ₐ is H, R₃ₐ is OH, Xₐ is O, R₁ₐ and R_{1b} are OH). A preferred compound of formula IVb is the Compound C-phosphate (R₂ₐ is H, R_{3b} is OH, Xₐ is O, R₁ₐ and R_{1b} are OH, Yₐ is O and R₄ₐ is heptyl). A preferred compound of formula V is Compound B-phosphate.

A preferred compound of formula V is phosphoric acid mono-[(R)-2-amino-2-methyl-4-(4-pentyloxy-phenyl)-butyl]ester.

A preferred compound of formula VIII is (2R)-2-amino-4-[3-(4-cyclohexyloxybutyl)benzo[b]thien-6-yl]-2-methylbutan-1-ol.

When the compounds of formulae I to IX have one or more asymmetric centers in the molecule, the present invention is to be understood as embracing the various optical isomers, as well as racemates, diastereoisomers and mixtures thereof are embraced. Compounds of formula III or IVb, when the carbon atom bearing the amino group is asymmetric, have preferably the R-configuration at this carbon atom.

Examples of pharmaceutically acceptable salts of the compounds of the formulae I to IX include salts with inorganic acids, such as hydrochloride, hydrobromide and sulfate, salts with organic acids, such as acetate, fumarate, maleate, benzoate, citrate, malate, methanesulfonate and benzenesulfonate salts, or, when appropriate, salts with metals such as sodium, potassium, calcium and aluminium, salts with amines, such as triethylamine and salts with dibasic amino acids, such as lysine. The compounds and salts of the methods of the present invention encompass hydrate and solvate forms.

The co-agent b) may be selected from the following groups of compounds:
i) Interferons, e.g. pegylated or non-pegylated α-interferons, or β-interferons or τ-interferons, e.g. administered by subcutaneous, intramuscular or oral routes, preferably β-interferons;
ii) An altered peptide ligand such as Glatiramer, e.g. in the acetate form;
iii) Immunosuppressants with optionally antiproliferative/antineoplastic activity, e.g. mitoxantrone, methotrexate, azathioprine, cyclophosphamide, or steroids, e.g. methylprednisolone, prednisone or dexamethasone, or steroid-secreting agents, e.g. ACTH;
iv) Adenosine deaminase inhibitors, e.g. cladribine;
v) IV immunoglobulin G (e.g. as disclosed in Neurology, 1998, May 50(5):1273-81
vi) Monoclonal antibodies to various T-cell surface markers, e.g. natalizumab (ANTEGREN®) or alemtuzumab;
vii) TH2 promoting cytokines, e.g. IL-4, IL-10, or compounds which inhibit expression of TH1 promoting cytokines, e.g. phosphodiesterase inhibitors, e.g. pentoxifylline;
viii) Antispasticity agents including baclofen, diazepam, piracetam, dantrolene, lamotrigine, rifluzole, tizanidine, clonidine, beta blockers, cyproheptadine, orphenadrine or cannabinoids;
ix) AMPA glutamate receptor antagonists, e.g. 2,3-dihydroxy-6-nitro-7-sulfamoylbenzo(f)quinoxaline, [1,2,3,4,-tetrahydro-7-morpholin-yl-2,3-dioxo-6-(trifluoromethyl)quinoxalin-1-yl]methylphosphonate, 1-(4-aminophenyl)-4-methyl-7,8-methylene-dioxy-5H-2,3-benzodiazepine, or (-)1-(4-aminophenyl)-4-methyl-7,8-methylene-dioxy-4,5-dihydro-3-methylcarbamoyl-2,3-benzodiazepine;
x) Inhibitors of VCAM-1 expression or antagonists of its ligand, e.g. antagonists of the α4β1 integrin VLA-4 and/or alpha-4-beta-7 integrins, e.g. natalizumab (ANTEGREN®);
xi) Anti-Macrophage migration inhibitory factor (Anti-MIF);
xii) Cathepsin S inhibitors;
xiii) mTor inhibitors.

Cathepsin S inhibitors include e.g.:
a) a compound as disclosed in WO 03/20721, e.g. a compound of formula: 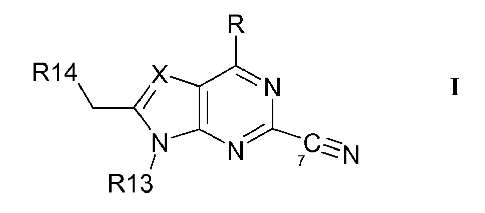 wherein
   R is H, -R2, -OR2 or NR1R2,
   wherein R1 is H, lower alkyl or C₃ to C₁₀ cycloalkyl, and
   R2 is lower alkyl or C₃ to C₁₀ cycloalkyl, and
   wherein each of R1 and R2 independently, is optionally substituted by halo, hydroxy, lower alkoxy, CN, NO₂, or optionally mono- or di-lower alkyl substituted amino;
   X is =N- or =C(Z)-,
   wherein Z is H, -C(O)-NR3R4, -NH-C(O)-R3, -CH₂-NH-C(O)-R3, -C(O)-R3, -S(O)-R3, -S(O)₂-R3,-CH₂-C(O)-R3, -CH₂-NR3R4, -R4, -C≡C-CH₂-R5, N-heterocyclyl, N-heterocyclyl-carbonyl, or -C(P)=C(Q)-R4
   wherein
   each of P and Q, independently, is H, lower alkyl or aryl,
   R3 is aryl, aryl-lower alkyl, C₃-C₁₀cycloalkyl, C₃-C₁₀cycloalkyl-lower alkyl, heterocyclyl or heterocyclyl-lower alkyl,
   R4 is H, aryl, aryl-lower alkyl, aryl-lower-alkenyl, C₃-C₁₀cycloalkyl, C₃-C₁₀cycloalkyllower alkyl, heterocyclyl or heterocyclyl-lower alkyl, or
   wherein R3 and R4 together with the nitrogen atom to which they are joined to form an N-heterocyclyl group,
   wherein N-heterocyclyl denotes a saturated, partially unsaturated or aromatic nitrogen containing heterocyclic moiety attached via a nitrogen atom thereof having from 3 to 8 ring atoms optionally containing a further 1, 2 or 3 heteroatoms selected from N, NR6, O, S, S(O) or S(O)₂ wherein R6 is H or optionally substituted (lower alkyl, carboxy, acyl (including both lower alkyl acyl, e.g. formyl, acetyl or propionyl, or aryl acyl, e.g. benzoyl), amido, aryl, S(O) or S(O)₂), and wherein the N-heterocyclyl is optionally fused in a bicyclic structure, e.g. with a benzene or pyridine ring, and wherein the N-heterocyclyl is optionally linked in a spiro structure with a 3 to 8 membered cycloalkyl or heterocyclic ring wherein the heterocyclic ring has from 3 to 10 ring members and contains from 1 to 3 heteroatoms selected from N, NR6, O, S, S(O) or S(O)₂ wherein R6 is as defined above), and
   wherein heterocyclyl denotes a ring having from 3 to 10 ring members and containing from 1 to 3 heteroatoms selected from N, NR6, O, S, S(O) or S(O)₂ wherein R6 is as defined above), and
   wherein each of R3 and R4, independently, is optionally substituted by one or more groups, e.g. 1-3 groups, selected from halo, hydroxy, oxo, lower alkoxy, CN or NO₂, or optionally substituted (optionally mono- or di-lower alkyl substituted amino, aryl, aryl-lower alkyl, N-heterocyclyl or N-heterocyclyl-lower alkyl (wherein the optional substitution comprises from 1 to 3 substituents selected from halo, hydroxy, lower alkoxy, CN, NO₂, or optionally mono- or di-lower alkyl substituted amino)), and wherein
   R5 is aryl, aryl-lower alkyl, aryloxy, aroyl or N-heterocyclyl as defined above, and
   wherein R5 is optionally substituted by R7 which represents from 1 to 5 substitutents selected from halo, hydroxy, CN, NO₂ or oxo, or optionally substituted (lower-alkoxy, lower-alkyl, aryl, aryloxy, aroyl, lower-alkylsulphonyl, arylsulphonyl, optionally mono- or di-lower alkyl substituted amino, or N-heterocyclyl, or N-heterocyclyl-lower alkyl (wherein N-heterocyclyl is as defined above), and
   wherein R7 is optionally substituted by from 1 to 3 substitutents selected from halo, hydroxy, optionally mono- or di- lower-alkyl substituted amino, lower-alkyl carbonyl, lower-alkoxy or lower-alkylamido;
   R13 is lower alkyl, C3 to C10 cycloalkyl or C3-C10cycloalkyl-lower alkyl, all of which are independently optionally substituted by halo, hydroxy, CN, NO2 or optionally mono- or di-lower alkyl-substituted amino; and
   R14 is H or optionally substituted (aryl, aryl-W-, aryl-lower alkyl-W-, C3 to C10 cycloalkyl, C3 to C10 cycloalkyl-W-, N-heterocyclyl or N-heterocyclyl-W- (wherein N-heterocyclyl is as defined above), phthalimide, hydantoin, oxazolidinone, or 2,6-dioxo-piperazine),
   wherein -W- is -O-, -C(O)-, -NH(R6)-, -NH(R6)-C(O)-, -NH(R6)-C(O)-O-, (where R6 is as defined above),-S(O)-, -S(O)₂- or -S-,
   wherein R14 is optionally substituted by R18 which represents from 1 to 10 substitutents selected from halo, hydroxy, CN, NO₂, oxo, amido, carbonyl, sulphonamido, lower-alkyldioxymethylene, or optionally substituted (lower-alkoxy, lower-alkyl, lower-alkenyl, lower alkynyl, lower alkoxy carbonyl, optionally mono- or di-lower alkyl substituted amino, aryl, aryl-lower alkyl, aryl-lower alkenyl, aryloxy, aroyl, lower-alkylsulphonyl, arylsulphonyl, N-heterocyclyl, N-heterocyclyl-lower alkyl (wherein N-heterocyclyl is as defined above), heterocyclyl or R14 comprising aryl has aryl fused with a hetero-atom containing ring, and
   wherein R18 is optionally substituted by R19 which represents from 1 to 4 substitutents selected from halo, hydroxy, CN, NO₂ or oxo, or optionally substituted (lower-alkoxy, lower-alkyl, lower-alkoxy-lower-alkyl, C₃-C₁₀cycloalkyl, lower-alkoxy carbonyl, halo-lower alkyl, optionally mono- or di-lower alkyl substituted amino, aryl, aryloxy, aroyl (e.g. benzoyl), acyl (e.g. lower-alkyl carbonyl), lower-alkylsulphonyl, arylsulphonyl or N-heterocyclyl, or N-heterocyclyl-lower alkyl (wherein N-heterocyclyl is as defined above)),
   wherein R19 is optionally substituted by from 1 to 4 substitutents selected from halo, hydroxy, CN, NO₂, oxo, optionally mono- or di-lower alkyl substituted amino, lower-alkyl, or lower-alkoxy;
b) a compound as disclosed in WO 00/69855, e.g. N2-(3-furanylcarbonyl)-L-norleucine-2(S)-methyl-4-oxotetrahydrofuran-3(R)-yl amide;
c) a compound as disclosed in WO 01/19796, WO 01/19808, WO 02/51983, WO 03/24923, WO 03/24924, WO 03/41649 or WO 03/42197, e.g. N-(2-(1-cyanocyclopropylamino)-1(R)-(2-benzylsulfonylmethyl)-2-oxoethyl)morpholine-4-carboxamide, N-(2-(cyanomethylamino)-1-(2-(difluoromethoxy)benzylsulfonylmethyl)-2-oxoethyl)pyridine-4-carboxamide, N-(2-(cyanomethylamino)-1(R)-(2-(difluoromethoxy)benzylsulfonylmethyl)-2-oxoethyl)-3,4-difluorobenzamide, N-(2-(cyanomethylamino)-1(R)-(2-(difluoromethoxy)benzylsulfonylmethyl)-2-oxoethyl)-3-methylbenzamide, N-(2-(cyanomethylamino)-1(R)-(2-(difluoromethoxy)benzylsulfonylmethyl)-2-oxoethyl)-1H-indole-5-carboxamide, N-(2-(cyanomethylamino)-1(R)-(2-(difluoromethoxy)benzylsulfonylmethyl)-2-oxoethyl)-5-methylthiophene-2-carboxamide, N-(2-(4-cyano-1-methylpiperidin-4-ylamino)-1(R)-(2-(difluoromethoxy)benzylsulfonylmethyl)-2-oxoethyl)morpholine-4-carboxamide, N-(2-(cyanomethylamino)-1(R)-(2-(difluoromethoxy)benzylsulfonylmethyl)-2-oxoethyl)-4-fluorobenzamide, N-(2-(cyanomethylamino)-1(R)-(2-(difluoromethoxy)benzylsulfonylmethyl)-2-oxoethyl)thiophene-3-carboxamide, N-(2-(cyanomethylamino)-1 (R)-(2-(difluoromethoxy)benzylsulfonylmethyl)-2-oxoethyl)thiophene-2-carboxamide or N-(2-(cyanomethylamino)-1(R)-(2-(difluoromethoxy)benzylsulfonylmethyl)-2-oxoethyl)morpholine-4-carboxamide;
d) a compound as disclosed in WO 00/51998, WO 03/29200 or WO 03/37892, e.g. N-(1(S)-(N-(2-(benzyloxy)-1(R)-cyanoethyl)carbamoyl)-2-cyclohexylethyl)morpholine-4-carboxamide;
e) a compound as disclosed in WO 02/14314, WO 02/14315 or WO 02/14317, e.g. N1-(3-chloro-2-(4-(2-hydroxy-3-(5-(methylsulfonyl)-3-(4-(trifluoromethyl)phenyl)-4,5,6,7-tetrahydro-1H-pyrazolo(4,3-pyhdin-1-yl)propyl)plperazin-1-yl)phenyl)-N3-methylurea, 1-(1-(3-(3-(4-bromophenyl)-5-(methylsulfonyl)-4,5,6,7-tetrahydro- H-pyrazolo(4,3-c)pyridine-1-yl)-2-hydroxypropyl)piperidin-4-yl)-6-chloro-1,2,3,4-tetrahydroquinolin-2-one, or 1-(5-(methylsulfonyl)-3-(4-(trifluoromethyl)phenyl-4,5,6,7-tetrahydro-1H-pyrazolo(4,3-c)pyridine-1-yl)-3-(4-(6-(4-morpholinyl)-1H-pyrrolo(3,2-c)pyridine-3-yl)piperidin-1-yl)propan-2-ol;
f) a compound as disclosed in WO 01/89451, e.g. 5-(2-morpholin-4ylethoxy)benzofuran-2-carboxylic acid ((S)-3-methyl-1-((S)-3-oxo-1-(2-(3-pyridin-2-ylphenyl)-acetyl)azepan-4-ylcarbamoyl)butylamide;
g) a compound as disclosed in WO 02/32879, WO 01/09169 or WO 00/59881A1, e.g. N-(1-benzothien-2-ylcarbonyl)-N-(2-(2-fluorophenyl)-4-oxo-1,2,3,4-tetrahydropyrimidin-5-yl)-L-leucinamide;
h) a compound as disclosed in WO 00/48992, WO 00/49007 or WO 00/49008.

The term "mTOR inhibitor" as used herein includes, but is not limited to rapamycin (sirolimus) or a derivative thereof. Rapamycin is a known macrolide antibiotic produced by Streptomyces hygroscopicus. Suitable derivatives of rapamycin include e.g. compounds of formula A 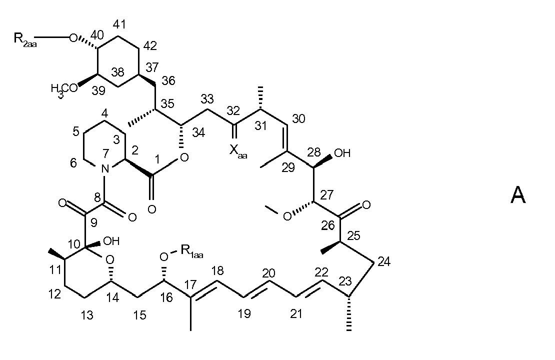 wherein
R₁ₐₐ is CH₃ or C₃₋₆alkynyl,
R₂ₐₐ is H or -CH₂-CH₂-OH, 3-hydroxy-2-(hydroxymethyl)-2-methylpropanoyl or tetrazolyl, and
Xₐₐ is =O, (H,H) or (H,OH)
provided that R₂ₐₐ is other than H when Xₐₐ is =O and R₁ₐₐ is CH₃.
or a prodrug thereof when R₂ₐₐ is -CH₂-CH₂-OH, e.g. a physiologically hydrolysable ether thereof.

Compounds of formula A are disclosed e.g. in WO 94/09010, WO 95/16691, WO 96/41807, USP 5,362,718 or WO 99/15530 which are incorporated herein by reference. They may be prepared as diclosed or by analogy to the procedures described in these references.

Preferred rapamycin derivatives are 32-deoxorapamycin, 16-pent-2-ynyloxy-32-deoxorapamycin, 16-pent-2-ynyloxy-32(S)-dihydro-rapamycin, 16-pent-2-ynyloxy-32(S)-dihydro-40-O-(2-hydroxyethyl)-rapamycin and, more preferably, 40-0-(2-hydroxyethyl)-rapamycin. Further examples of rapamycin derivatives include e.g. CCI779 or 40- [3-hydroxy-2-(hydroxymethyl)-2-methylpropanoate]-rapamycin or a pharmaceutically acceptable salt thereof, as disclosed in USP 5,362,718, ABT578 or 40-(tetrazolyl)-rapamycin, particularly 40-epi-(tetrazolyl)-rapamycin, e.g. as disclosed in WO 99/15530, or rapalogs as disclosed e.g. in WO 98/02441 and WO01/14387, e.g. AP23573 or TAFA-93.

In each case where citations of patent applications or scientific publications are given, the subject-matter relating to the compounds is hereby incorporated into the present application by reference. Comprised are likewise the pharmaceutically acceptable salts thereof, the corresponding racemates, diastereoisomers, enantiomers, tautomers as well as the corresponding crystal modifications of above disclosed compounds where present, e.g. solvates, hydrates and polymorphs, which are disclosed therein. The compounds used as active ingredients in the combinations of the invention can be prepared and administered as described in the cited documents, respectively. Also within the scope of this invention is the combination of more than two separate active ingredients as set forth above, i.e. a pharmaceutical combination within the scope of this invention could include three active ingredients or more. Further both the first agent and the co-agent are not the identical ingredient.

Utility of the compounds of formula I in treating demyelinating diseases, e.g. multiple sclerosis or Guillain-Barré syndrome as hereinabove specified, may be demonstrated in animal test methods as well as in clinic, for example in accordance with the methods hereinafter described. The most widely used animal model for multiple sclerosis is experimental autoimmune encephalomyelitis (EAE), based on shared histopathological and clinical features with the human disease.

### A.1 In vivo: SJL/J Mouse model of chronic progressive EAE

The disease course in this animal model shares some common features with SP-and PR-MS. Immunization: On day 0 female SJL/J mice are immunized (subcutaneous flank injection) with 200 µl inoculum containing 500 µg bovine myelin basic protein (MBP) emulsified in complete Freund's adjuvant (CFA). On day 9 mice are boosted by a second MBP injection and an additional intravenous adjuvant injection consisting of 200 ng *B. pertussis* toxin. A final Pertussis injection is given on day 11.

Most of the MBP-immunized mice exhibit a severe bout of EAE by day 21. This is followed by a recovery phase starting around day 25, during which time mice remain symptom-free for about 20 days. Subsequently, by days 45-47, approximately 50% of the animals go into the progressive phase of the disease. Therefore, therapeutic treatment with test compounds starts on day 21 when the disease is fully established and continues until day 70, unless stated otherwise. Recombinant mouse interferon β (INFβ Calbiochem/Biosciences) is dissolved in saline and given by intraperitoneal injection 3x per week. Compound (a), e.g. Compound A or B, is diluted in water and given p.o. 5x per week by gavage. Mice in the vehicle control group are MBP-immunized and treated with water.

Each experimental group consists of 10 mice, which are examined daily for clinical EAE symptoms. Disease incidence and the day of EAE onset also are recorded. Clinical grades of EAE are assessed using a scale from 0 to 3. Any disease-related mortality which occurs after starting drug treatment is recorded with a maximum score of 3.

Compound (a), e.g. Compound A or B at 0.6 mg/kg p.o. in combination with INFβ (10 000 IU) prevents disease progression for one month (days 45-75), compared to the vehicle-treated controls. In contrast, administration of INFβ alone (10 000 IU 3x/week) only marginally inhibits disease progression for about 1 week, after which the mice went on to develop a full EAE response that is indistinguishable from the disease course in vehicle-treated controls by day 68 onwards.

### A.2 In vivo: Optic Neuritis in the DA rat model of chronic-protracted EAE

Ocular pathologic manifestations such as optic neuritis (neuromyelitis optica) are frequent in multiple sclerosis and often precede or accompany plaque formation in the brain white matter. Ocular areas, especially the optic chiasma, also are important targets in demyelinating forms of EAE. In such EAE models, functional disability caused by demyelination of the optic nerve can be assessed by electrophysiological methods, such as visual evoked cortical potentials and electroretinogram, in conjunction with morphological analysis of the ocular tissue.

Immunization: On day 0, female DA rats are immunized by a single intradermal injection at the tail base with 100-200 µl inoculum containing a recombinant encephalitogenic peptide, e.g. myelin oligodendrocyte glycoprotein, or a homogenate of syngeneic central nervous system tissue emulsified in one part CFA (volume:volume). Neurologic symptoms develop by 10 days post-immunization, and clinical grades of EAE are assessed using a scale of 0 to 4. Therapeutic treatment with the test compound starts when the disease is fully established, usually on day 12, and continues for 2 weeks. Compound (a), e.g. Compound A or B at 0.3 mg/kg p.o. given once a day for 2 weeks, prevents disease progression for at least 2 months, compared to the vehicle-treated controls. Using combination treatment, suboptimal doses of Compound A or B (<0.1 mg/kg p.o.) and a mTOR inhibitor (<1 mg/kg p.o.) also curtail development of EAE symptoms and prevent disease-related weight loss after therapeutic dosing in the DA rat model. In a prophylactic treatment regimen, similar combinations of Compound A or B and a mTOR inhibitor prevent disease onset in the Lewis rat model of EAE, induced by an intradermal injection of guinea pig neuroantigen.

### B Clinical Trial

Suitable clinical studies are, for example, open label, dose escalation studies in patients with multiple sclerosis. Such studies prove in particular the synergism of the active ingredients of the combination of the invention. The beneficial effects on multiple sclerosis can be determined directly through the results of these studies which are known as such to a person skilled in the art. Such studies are, in particular, suitable to compare the effects of a monotherapy using the active ingredients and a combination of the invention. Preferably, the dose of agent (a) is escalated until the Maximum Tolerated Dosage is reached, and the co-agent (b) is administered with a fixed dose. Alternatively, the agent (a) is administered in a fixed dose and the dose of co-agent (b) is escalated. Each patient receives doses of the agent (a) either daily or intermittent. The efficacy of the treatment can be determined in such studies, e.g., after 12, 18 or 24 weeks by evaluation of symptom scores every 6 weeks.

Alternatively, a placebo-controlled, double blind study can be used in order to prove the benefits of the combination of the invention mentioned herein.

The administration of a pharmaceutical combination of the invention results not only in a beneficial effect, e.g. a synergistic therapeutic effect, e.g. with regard to alleviating, delaying progression of or inhibiting the symptoms, but also in further surprising beneficial effects, e.g. fewer side-effects, an improved quality of life or a decreased morbidity, compared with a monotherapy applying only one of the pharmaceutically active ingredients used in the combination of the invention.

A further benefit is that lower doses of the active ingredients of the combination of the invention can be used, for example, that the dosages need not only often be smaller but are also applied less frequently, which may diminish the incidence or severity of side-effects. This is in accordance with the desires and requirements of the patients to be treated.

The terms "co-administration" or "combined administration" or the like as utilized herein are meant to encompass administration of the selected therapeutic agents to a single patient, and are intended to include treatment regimens in which the agents are not necessarily administered by the same route of administration or at the same time.

It is one objective of this invention to provide a pharmaceutical composition comprising a quantity, which is jointly therapeutically effective against multiple sclerosis or disorders associated therewith comprising a combination of the invention. In this composition, the first agent a) and co-agent (b) may be administered together, one after the other or separately in one combined unit dosage form or in two separate unit dosage forms. The unit dosage form may also be a fixed combination.

The pharmaceutical compositions for separate administration of the first agent a) and co-agent b) or for the administration in a fixed combination, i.e. a single galenical composition comprising at least two combination partners a) and b), according to the invention may be prepared in a manner known per se and are those suitable for enteral, such as oral or rectal, and parenteral administration to mammals (warm-blooded animals), including humans, comprising a therapeutically effective amount of at least one pharmacologically active combination partner alone, e.g. as indicated above, or in combination with one or more pharmaceutically acceptable carriers or diluents, especially suitable for enteral or parenteral application.

Suitable pharmaceutical compositions contain, for example, from about 0.1 % to about 99.9%, preferably from about 1 % to about 60 %, of the active ingredient(s). Pharmaceutical preparations for the combination therapy for enteral or parenteral administration are, for example, those in unit dosage forms, such as sugar-coated tablets, tablets, capsules or suppositories, or ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

In particular, a therapeutically effective amount of each of the combination partner of the combination of the invention may be administered simultaneously or sequentially and in any order, and the components may be administered separately or as a fixed combination. For example, the method of delay of progression or treatment of multiple sclerosis or disorders associated therewith according to the invention may comprise (i) administration of the first agent a) in free or pharmaceutically acceptable salt form and (ii) administration of a co-agent b) in free or pharmaceutically acceptable salt form, simultaneously or sequentially in any order, in jointly therapeutically effective amounts, preferably in synergistically effective amounts, e.g. in daily or intermittently dosages corresponding to the amounts described herein. The individual combination partners of the combination of the invention may be administered separately at different times during the course of therapy or concurrently in divided or single combination forms. Furthermore, the term administering also encompasses the use of a pro-drug of a combination partner that convert *in vivo* to the combination partner as such. The instant invention is therefore to be understood as embracing all such regimens of simultaneous or alternating treatment and the term "administering" is to be interpreted accordingly.

The effective dosage of each of the combination partners employed in the combination of the invention may vary depending on the particular compound or pharmaceutical composition employed, the mode of administration, the condition being treated, the severity of the condition being treated. Thus, the dosage regimen of the combination of the invention is selected in accordance with a variety of factors including the route of administration and the renal and hepatic function of the patient. A physician, clinician or veterinarian of ordinary skill can readily determine and prescribe the effective amount of the single active ingredients required to alleviate, counter or arrest the progress of the condition. Optimal precision in achieving concentration of the active ingredients within the range that yields efficacy without toxicity requires a regimen based on the kinetics of the active ingredients' availability to target sites, particularly when co-agent b) is a small molecule.

Daily dosages for the first agent a) will, of course, vary depending on a variety of factors, for example the compound chosen, the particular condition to be treated and the desired effect. In general, however, satisfactory results are achieved on administration of agent a) at daily dosage rates of the order of ca. 0.03 to 2.5 mg/kg per day, particularly 0.1 to 2.5 mg/kg per day, e.g. 0.5 to 2.5 mg/kg per day, as a single dose or in divided doses. The S1 P receptor agonist, e.g. a compound of formulae I to VII, e.g. Compound A or B, may be administered by any conventional route, in particular enterally, e.g. orally, e.g. in the form of tablets, capsules, drink solutions or parenterally, e.g. in the form of injectable solutions or suspensions. Suitable unit dosage forms for oral administration comprise from ca. 0.02 to 50 mg active ingredient, usually 0.1 to 30 mg, e.g. Compound A or B, together with one or more pharmaceutically acceptable diluents or carriers therefor. These dosages are also indicated when the S1 P receptor agonist is used alone in the treatment of optic neuritis.

Interferons may be administered to a human in the following dosage ranges: interferon β-1b: up to 0.25 mg sc; interferon β-1a: up to 30 µg im; interferon α-2a: up to 10 million I.U. (MIU) sc or up to 1 MIU orally; interferon α-2b: up to 10 MIU sc or up to 1 MIU orally; pegylated interferon α-2a: up to 270 µg sc; pegylated interferon α-2b: up to 2.0 µg/kg sc.

Glatiramer may be administered to a human in a dosage range up to 20 mg sc, or up to 50 mg po.

Antineoplastic/antiproliferative immunosuppressants may be administered to a human in the forllowing dosage ranges: cyclophosphamide 500-1500 mg/m² IV; methotrexate up to 20 mg po; mitoxantrone 12 mg/m² IV, or azathioprine 2 mg/kg po.

Steroids may be administered to a human in the following dosage ranges: methylprednisolone 1-2-mg IV, or 24-48 mg po; prednisone 1 mg/kg po, or ACTH up to 100 MIU.

ADA inhibitors such as cladribine may be administered to a human in a dosage range up to 0.07 mg/kg/day.

IV Immunoglobulin G may be administered in a human in a dosage range up to 400 mg/kg IV.

Monoclonal antibodies to various T-cell surface markers may be administered in a human in the following dosage ranges: natalizumab up to 3mg/kg IV, alemtuzumab up to 30 mg sc or IV.

TH2 promoting cytokines may be administered to a human in the following dosage ranges: IL-4 up to 3µg/kg sc, or IL-10 up to 20µg/kg sc. Compounds which inhibit expression of TH1 promoting cytokines such as the phosphodiesterase inhibitor pentoxifylline may be administered in a human in a dosage range up to 4 mg po.

Antispasticity agents may be administered in a human in the following doage ranges: baclofen up to 100 mg po, diazepam up to 20 mg po, piracetam up to 24 mg po, dantrolene up to 100 mg po, lamotrigine up to 100 mg/day, riluzole up to 100 mg po, tizanidine up to 12 mg po, clonidine up to 0.1 mg po, β blockers (e.g. propanolol) up to 160 mg po, cyproheptadine up to 8 mg po, orphenadrine up to 100 mg po and cannabinoids ( e.g. dronabinol) up to 5 mg po.

Cathepsin S inhibitors, e.g. a compound as disclosed in WO 03/20721, may be administered to a human in the dosage range 0.1 to 100 mg/kg/day.

mTor inhibitors, e.g. rapamycin or a derivative thereof, e.g. 40-O-(2-hydroxyethyl)-rapamycin, may be administered in a dosage range varying from about 0.1 to 25 mg/kg/day.

When used in treating, alleviating or delaying progression of optic neuritis, the S1 P receptor agonist, e.g. a compound of formula I to VII, e.g. a compound A or B, may be administered systematically or topically, by any conventional route, in particular enterally, e.g. orally, e.g. in the form of tablets or capsules, topically, e.g. in the form of a topical ophthalmic composition, e.g. comprising an ophthalmic carrier. Pharmaceutical compositions comprising an S1 P receptor agonist in association with at least one pharmaceutically acceptable carrier or diluent may be manufactured in conventional manner, e.g. by mixing the ingredients.

Compounds of formulae I to VII are well tolerated at dosages required for use in accordance with the present invention. For example, the acute LD₅₀ is >10 mg/kg p.o. in rats and monkeys for Compound A.

## Claims

1. A pharmaceutical combination for treating, alleviating or delaying progression of the symptoms of a demyelinating disease comprising:
a) a sphingosine-1-phosphate (S1P) receptor agonist, and
b) at least one co-agent shown to have clinical activity against at least one symptom of a demyelinating disease,

2. Combination according to claim 1, wherein the co-agent b) is selected from the group consisting of interferons, altered peptide ligands, immunosuppressants, adenosine deaminase inhibitors, IV immunoglobulin G, monoclonal antibodies to T-cell surface markers, TH2 promoting cytokines, compounds which inhibit expression of TH1 promoting cytokines, antispasticity agents, AMPA glutamate receptor antagonists, inhibitors of VCAM-1 expression or antagonists of its ligand, anti-macrophage migration inhibitory factor, cathepsin S inhibitors and mTOR inhibitors.

3. Combination according to claim 1, wherein the co-agent b) is an interferon.

4. Combination according to claim 1, wherein the co-agent b) is a β interferon.

5. Combination according to claim 1, wherein the co-agent b) is an mTor inhibitor.

6. Combination according to claim 1, wherein the co-agent b) is rapamycin (sirolimus) or a derivative thereof.

7. Combination according to claim 1, wherein the co-agent b) is 40-0-(2-hydroxyethyl)-rapamycin.

8. Combination according to any preceding claims, wherein the S1 P receptor agonist is agonist is selected from 2-amino-2-[2-(4-octylphenyl) ethyl]propane-1 ,3-diol (FTY720), a pharmaceutically acceptable salt thereof, and FTY720-phosphate.

9. Combination according to any one of claims 1 to 7, wherein the S1 P receptor agonist is 2-amino-2-[2-(4-octylphenyl) ethyl]propane-1 ,3-diol (FTY720), a pharmaceutically acceptable salt thereof

10. Combination according to any one of claims 1 to 7, wherein the S1 P receptor agonist is 2-amino-2-[2-(4-octylphenyl) ethyl]propane-1 ,3-diol hydrochloride.

11. Combination according to any preceding claims for treating, alleviating or delaying the progression of multiple sclerosis or Guillain-Barré syndrome.
